# EUROPÄISCHE PATENTSCHRIFT

(11) **EP 0 857 461 B1**
(45) Veröffentlichungstag und Bekanntmachung des Hinweises auf die Patenterteilung: **28.07.2004**
(21) Anmeldenummer: 98200136.4
(22) Anmeldetag: 20.01.1998
(51) Int. Cl.: A61B 6/00, A61B 6/08, A61B 6/12

(54) **Verfahren und Anordnung zur Positionsbestimmung bei der Röntgenbildgebung**
Method and system for position determination during X-ray imaging
Procédé et système pour déterminer la position pendant l'imagerie radiologique

(30) Priorität: 31.01.1997 DE 19703556
(43) Veröffentlichungstag der Anmeldung: 12.08.1998
(73) Patentinhaber: Philips Intellectual Property & Standards GmbH, 20099 Hamburg (DE); Koninklijke Philips Electronics N.V., 5621 BA Eindhoven (NL)
(72) Erfinder: Schmitz, Georg, Dr., Röntgenstrasse 24, 22335 Hamburg (DE); Sabczynski, Jörg, Dr., Röntgenstrasse 24, 22335 Hamburg (DE)
(74) Vertreter: Volmer, Georg, Dipl.-Ing.

(56) Entgegenhaltungen:
- US-A- 5 389 101

## Beschreibung

Die Erfindung betrifft ein Verfahren zur Positionsbestimmung bei der Röntgenbildgebung sowie eine Anordnung zur Durchführung eines derartigen Verfahrens mit einem Röntgengerät, einer Detektoranordnung mit mindestens zwei Detektorelementen und mit einer Markeranordnung.

Eine Anordnung zur Positionsbestimmung bei der medizinischen Bildgebung ist in der PCT/IB 96/00384 (WO 97/40763) beschrieben. Zwei Kameras, die sichtbares Licht oder Infrarotlicht detektieren können, sind dort ortsfest im Raum an einem Stativ angeordnet. An einem Behandlungsinstrument sind Dioden, welche von den Kameras detektierbares Licht emittieren, angebracht. Mit den Kameras kann die Position des Behandlungsinstruments in einem mit den Kameras verkoppelten Koordinatensystem bestimmt werden. Diese Position kann mittels einer vorab bestimmten Transformationsmatrix in eine Bildposition umgerechnet werden, wobei das Bild vorher mittels Computertomographie (CT) oder Magnetresonanztomographie (MR) erfaßt wurde. Um die genannte Transformationsmatrix zur Umrechnung von Kamerakoordinaten in Bildkoordinaten zu bestimmen, werden am Patienten während der Bilderfassung Marker angebracht, die ebenfalls abgebildet werden und im Bild sichtbar sind. Diese Marker werden anschließend mit dem mit Dioden versehenen Behandlungsinstrument oder mit einem separaten, mit Dioden versehenen Zeigeinstrument angefahren, wodurch deren Position in Kamerakoordinaten erfaßt wird. Damit ist die erforderliche Verbindung zwischen Kamerakoordinaten und Bildkoordinaten hergestellt und die Position des Behandlungsinstruments während einer Behandlung im Bild kann angezeigt werden.

Eine derartige Anordnung ist auch im US Patent 5,389,101 (Heilbrun et al; Feb. 14, 1995) beschrieben.

Auch bei der konventionellen Röntgenbildgebung, insbesondere bei der intraoperativen Bildgebung kann die exakte Zuordnung eines Punktes in einem Röntgenbild zu einem Punkt in oder am Untersuchungsobjekt, beispielsweise einem Patienten, von großer Bedeutung sein. Auch die exakte Positionsbestimmung eines Behandlungsinstrumentes oder eines anderen medizinischen Gerätes, wie beispielsweise eines Strahlentherapiegerätes ist wünschenswert. Beim bekannten Verfahren ist erforderlich, daß am Untersuchungsobjekt über längere Zeit Marker an festen Positionen angebracht sind, nämlich bereits bei der Bilderfassung mittels CT oder MR und später bei der Positionserfassung mittels der Kameras. Auch die Anordnung der Kameras an einem ortsfesten Stativ ist für eine Positionsbestimmung bei der Röntgenbildgebung nicht ideal. Insbesondere bei der intraoperativen Röntgenbilderfassung mit einem schwenkbaren Röntgengerät würden bei einer solchen Anordnung der Kameras häufig die Sichtlinien der Kameras entweder durch das Röntgengerät selbst oder durch behandelnde Personen verdeckt werden.

Der Erfindung liegt deshalb die Aufgabe zugrunde, ein geeignetes Verfahren zur Positionsbestimmung bei der Röntgenbildgebung sowie eine Anordnung zur Durchführung eines derartigen Verfahrens anzugeben.

Die Aufgabe betreffend das Verfahren wird erfindungsgemäß dadurch gelöst, daß
- mit einem Röntgengerät mindestens ein Röntgenbild eines Untersuchungsobjektes erstellt wird und mit einer am Röntgengerät angeordneten Detektoranordnung die Position einer am Untersuchungsobjekt oder relativ zum Untersuchungsobjekt ortsfest angeordneten Markeranordnung in einem mit der Detektoranordnung verkoppelten Detektorkoordinatensystem erfaßt wird,
- die Position des Röntgengerätes in einem mit dem Untersuchungsobjekt verkoppelten Objektkoordinatensystem bestimmt wird
- und danach die Position eines als Bildpunkt in einem Röntgenbild abgebildeten Objektpunktes im Objektkoordinatensystem bestimmt wird.

Die Position der Markeranordnung wird in derselben Stellung des Röntgengeräts erfaßt, in der auch das Röntgenbild erstellt wurde. Diese beiden Vorgänge können also auch gleichzeitig erfolgen. Durch die Erfassung der Position der Markeranordnung im Detektorkoordinatensystem wird automatisch auch die Position der Detektoranordnung im Objektkoordinatensystem gewonnen, da die Markeranordnung ortsfest bezüglich des Untersuchungsobjekts angeordnet ist und das Objektkoordinatensystem fest mit dem Untersuchungsobjekt und somit auch fest mit der Markeranordnung verkoppelt ist. Diese automatische Gewinnung der Position der Markeranordnung in Objektkoordinaten aus der erfaßten Position in Detektorkoordinaten ist durch die Funktionalität des aus der Detektoranordnung und der Markeranordnung bestehenden Positionsmeßsystems gegeben.

Da die Detektoranordnung ortsfest am Röntgengerät angeordnet ist, kann aus der nun bekannten Position der Detektoranordnung im Objektkoordinatensystem leicht die Position des Röntgengerätes im Objektkoordinatensystem bestimmt werden. Bevorzugt werden bei der Bestimmung der Position des Röntgengerätes charakteristische Punkte wie die Lage des Fokuspunktes und die Lage der Abbildungsebene bestimmt. Anschließend kann daraus eine Zuordnung zwischen einem Bildpunkt in einem Röntgenbild und einem Objektpunkt des Untersuchungsobjektes im Objektkoordinatensystem erfolgen. Sofern nur ein einziges Röntgenbild erfaßt wurde, kann die Position eines zu einem Bildpunkt gehörigen Objektpunktes nur insoweit bestimmt werden, als einem Bildpunkt ein zwischen Fokuspunkt und Abbildungsebene des Röntgengerätes liegender Strahl zugeordnet werden kann. Erst bei mehreren, aus unterschiedlichen Positionen des Röntgengeräts erfaßten Röntgenbildern kann eine genauere Positionsbestimmung des Objektpunktes erfolgen.

In einer Ausgestaltung der Erfindung ist vorgesehen, daß mindestens zwei Röntgenbilder des Untersuchungsobjektes aus unterschiedlichen Richtungen erfaßt werden und daß gleichzeitig zur Röntgenbilderfassung die Position des Fokuspunktes und die Lage der Abbildungsebene des Röntgengerätes im Objektkoordinatensystem bestimmt werden. Bereits bei zwei Röntgenbildern kann die Position eines Objektpunktes, welcher in beiden Röntgenbildern als Bildpunkt abgebildet ist, im Objektkoordinatensystem exakt bestimmt werden. Beispielsweise genügen dazu zwei Röntgenbilder, welche aus senkrecht zueinander liegenden Richtungen erfaßt wurden.

Eine Weiterbildung dieses Verfahrens sieht erfindungsgemäß vor, daß die Position des Objektpunktes mittels zweier Geraden bestimmt wird, wobei die erste Gerade durch den zum Objektpunkt gehörigen Bildpunkt in einem ersten Röntgenbild und die erste Position des Fokuspunktes verläuft und die zweite Gerade durch den zum Objektpunkt gehörigen Bildpunkt in einem zweiten Röntgenbild und durch die zweite Position des Fokuspunktes verläuft, wobei sich der Objektpunkt als Schnittpunkt der beiden Geraden oder als Mittelpunkt der kürzesten Verbindungsstrecke der beiden Geraden ergibt. Der Objektpunkt läßt sich also sehr exakt durch einfache Berechnung des Schnittpunktes zweier Geraden oder durch Berechnung eines Punktes, der in der Mitte auf der kürzesten Verbindungsstrecke zwischen den Geraden liegt, ermitteln.

Eine weitere Ausgestaltung des erfindungsgemäßen Verfahrens sieht vor, daß mit der Detektoranordnung die Position eines mit einer weiteren Markeranordnung versehenen medizinischen Gerätes im Detektorkoordinatensystem erfaßt wird und daß aus der Position des medizinischen Gerätes im Detektorkoordinatensystem dessen Position im Bildkoordinatensystem eines Röntgenbildes bestimmt wird. Mittels der bereits zu Anfang bestimmten Umrechnungsvorschrift zwischen Detektorkoordinaten und Objektkoordinaten kann zunächst aus der erfaßten Position des medizinischen Gerätes im Detektorkoordinatensystem dessen Position im Objektkoordinatensystem und anschließend daraus dessen Position im Bildkoordinatensystem eines Röntgenbildes bestimmt werden. Dadurch ist es möglich, die Position des medizinischen Gerätes, das beispielsweise ein Behandlungsinstrument sein kann, unmittelbar im Röntgenbild einzublenden, so daß die behandelnde Person jederzeit weiß, an welcher Stelle am oder im Untersuchungsobjekt sich das Behandlungsinstrument gerade befindet, auch wenn Teile des Behandlungsinstruments von außen nicht sichtbar sind. Das medizinische Gerät kann beispielsweise auch ein Strahlentherapiegerät sein, dessen Position im Bildkoordinatensystem nach dem erfindungsgemäßen Verfahren exakt bestimmt werden kann, wodurch eine exakte Ausrichtung des Therapiestrahls auf die zu behandelnde Stelle möglich ist.

In einer davon ausgehenden Weiterbildung des erfindungsgemäßen Verfahrens ist vorgesehen, daß die Position des medizinischen Gerätes im Bildkoordinatensystem aus dem Schnittpunkt einer durch den Fokuspunkt und die Position des medizinischen Gerätes im Objektkoordinatensystem verlaufenden Gerade mit der Abbildungsebene berechnet wird. Da die Position des Röntgengerätes, insbesondere die Lage des Fokuspunktes und der Abbildungsebene des Röntgengerätes, und die Position des medizinischen Gerätes im Objektkoordinatensystem bei der Erfassung eines Rontgenbildes ermittelt werden, kann durch einfache Schnittpunktbestimmungen einer Gerade mit der Abbildungsebene die Position des medizinischen Gerätes im Bildkoordinatensystem bestimmt werden.

Die Aufgabe betreffend eine Anordnung zur Durchführung des Verfahrens wird erfindungsgemäß durch eine eingangs genannte Anordnung gelöst, die dadurch gekennzeichnet ist, daß die Detektoranordnung am Röntgengerät angeordnet ist und daß die Markeranordnung am Untersuchungsobjekt oder relativ zum Untersuchungsobjekt ortsfest angeordnet ist. Dadurch wird erreicht, daß der Sichtbereich der Detektoranordnung und der Durchleuchtungsbereich des Röntgengeräts im wesentlichen oder zumindest größtenteils übereinstimmen. Während bei der bekannten Anordnung, bei der die Detektoranordnung an einem Stativ angeordnet ist, der Sichtbereich der Detektoranordnung durch eine behandelnde Person oder ein Gerät verdeckt werden kann, wodurch eine Positionsbestimmung eines Objektpunktes oder eines Behandlungsinstrumentes bei Verwendung einer optischen Detektoranordnung verhindert wird, kann dies bei der erfindungsgemäßen Anordnung kaum noch auftreten. Während einer Röntgendurchleuchtung ist auf jeden Fall der Durchleuchtungsbereich frei von Behandlungsinstrumenten oder behandelnden Personen, so daß gleichzeitig eine Positionserfassung der am Untersuchungsobjekt angebrachten Markeranordnung mittels der Detektoranordnung möglich ist. Auch während der Zeit, in der keine Röntgendurchleuchtung stattfindet, ist in der Regel der Bereich zwischen Röntgengerät und Untersuchungsobjekt frei von anderen Geräten und behandelnden Personen, so daß auch wahrend dieser Zeit die Position von Behandlungsinstrumenten mittels der Detektoranordnung im Detektorkoordinatensystem erfaßt und anschließend im Bildkoordinatensystem bestimmt werden kann.

Eine Weiterbildung der erfindungsgemäßen Anordnung sieht vor, daß die Detektoranordnung am Röntgenstrahler oder an der Röntgenbildaufnahme- oder Röntgenbildverstärkereinrichtung angeordnet ist. Bei dieser Anordnung der Detektoranordnung ist die Gefahr, daß die Sichtlinien der Detektoranordnung unterbrochen werden könnten, sehr gering. Bei der Wahl der Lage der Detektoranordnung ist natürlich zu berücksichtigen, daß die Sichtlinien der Detektoranordnung in bestimmten Positionen des Röntgengeräts nicht durch Teile des Röntgengerätes selbst, wie beispielsweise den Patiententisch, verdeckt werden. Die Detektoranordnung ist deshalb bevorzugt an einem in den meisten Positionen des Röntgengeräts oberhalb des Untersuchungsobjektes befindlichen Teil des Röntgengeräts angeordnet.

Gemäß einer Weiterbildung der Erfindung ist vorgesehen, daß eine weitere Markeranordnung an einem medizinischen Gerät, insbesondere einem Behandlungsinstrument oder einer Strahlentherapieanordnung angeordnet ist. Die Erfassung der Position dieses medizinischen Geräts kann gleichzeitig zur Erfassung eines Röntgenbildes erfolgen, aber auch zu jedem sonstigen beliebigen Zeitpunkt. Voraussetzung ist jedoch immer, daß auch die aktuelle Position des Röntgengeräts mittels der Detektoranordnung erfaßt und bestimmt wurde, da diese Positionsdaten zur Bestimmung der Position des medizinischen Geräts im Bildkoordinatensystem erforderlich sind.

Bevorzugt ist erfindungsgemäß vorgesehen, daß die Markeranordnungen optische Lichtquellenmarker, beispielsweise Infrarotlicht oder sichtbares Licht emittierende Leuchtdioden, retroreflektierende Marker oder mit einem Erkennungsmuster versehene Marker aufweisen. Für die Detektoranordnung werden entsprechende optische Detektoren verwendet, die für die jeweilige Strahlung empfindlich sind. Bei der Verwendung retroreflektierender Marker ist außerdem in der Nähe der Detektoranordnung eine weitere Strahlungsquelle vorgesehen, deren Strahlung von den Markern reflektiert und dann von der Detektoranordnung detektiert wird, wodurch die Position der Marker bestimmbar ist. Bei der Verwendung von mit Erkennungsmustern versehenen Markern weist die Detektoranordnung entsprechende Detektoren auf, die aus der Erkennung derartiger Muster eine Position bestimmen können.

Eine Weiterbildung der erfindungsgemäßen Anordnung sieht außerdem vor, daß die Detektoranordnung zwei, jeweils eine zweidimensionale Position erfassende oder drei, jeweils eine eindimensionale Position erfassende optische Detektoren umfaßt, welche Infrarotlicht oder sichtbares Licht detektieren können, vorzugsweise Infrarot-CCD-Kameras, und daß die Markeranordnung mindestens jeweils drei Infrarotlicht oder sichtbares Licht emittierende Leuchtdioden umfassen. Dies stellt eine einfache und kostengünstige Lösung dar, mit der Positionen mit hoher Genauigkeit bestimmt werden können.

Eine alternative Ausgestaltung der erfindungsgemäßen Anordnung sieht vor, daß die Markeranordnung mindestens einen elektromagnetischen Sendeanordnung und die Detektorvorrichtung mindestens eine elektromagnetische Empfangsanordnung aufweist. Die elektromagnetische Sendeanordnung, die aufeinander senkrecht stehende elektromagnetische Felder erzeugen kann, und die elektromagnetische Empfangsanordnung, die elektromagnetische Felder detektieren kann, können anstelle der optischen Marker- und Detektoranordnung verwendet werden. Das Problem der Sichtlinienverdeckung durch Personal oder Geräte spielt bei Verwendung elektromagnetische Anordnungen, beispielsweise kleiner Spulenanordnungen, keine sehr große Rollen, wofür jedoch die optische Marker- und Detektoranordnung eine größere Reichweite aufweist.

Eine bevorzugte Ausgestaltung der Erfindung sieht vor, daß das Röntgengerät ein C-Bogen-Röntgengerät ist. Ein derartiges Röntgengerät wird häufig zur intraoperativen Röntgenbildgebung benutzt, um aus unterschiedlichen Positionen während der Operation Röntgenbilder vom Patienten zu erstellen. Da die Detektoranordnung fest am Röntgengerät angebracht ist, verändert sich bei einer Schwenkung des C-Bogens unmittelbar auch die Position der Detektoranordnung, während die Position der Markeranordnung fest am Patienten bzw. ortsfest relativ zum Patienten angeordnet ist. Als festes Bezugskoordinatensystem wird demnach das Koordinatensystem der Markeranordnung (das Objektkoordinatensystem) benutzt.

Die Erfindung wird nachfolgend anhand der Zeichnungen näher erläutert. Es zeigen:
- Fig. 1: eine schematische Darstellung einer erfindungsgemäßen Anordnung mit einem C-Bogen-Röntgengerät,
- Fig. 2: einen Ablaufplan des erfindungsgemäßen Verfahrens,
- Fig. 3: eine schematische Darstellung zur Erläuterung der Positionsbestimmung nach dem erfindungsgemäßen Verfahren und
- Fig. 4: einen weiteren Ablaufplan zur Erläuterung einer Ausführungsform des erfindungsgemäßen Verfahrens.

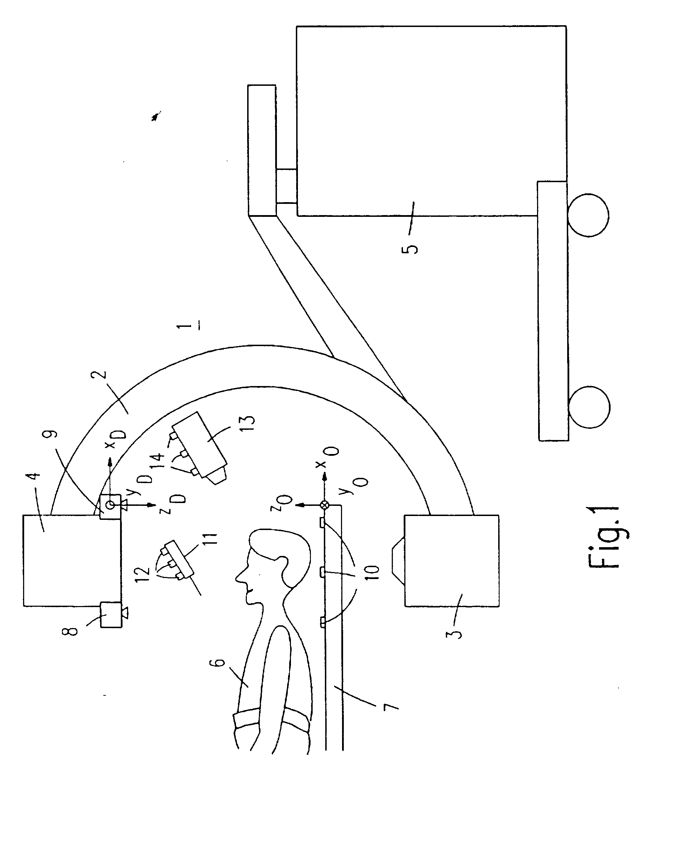

In Fig. 1 ist eine erfindungsgemäße Anordnung mit einem C-Bogen-Röntgengerät 1 dargestellt. Am unteren Ende des C-Bogens 2 befindet sich ein Röntgenstrahler 3, am oberen Ende des C-Bogens 2 befindet sich eine Bildaufnahme- und Bildverstärkereinrichtung 4. Die Elektronik zur Steuerung und Datenverarbeitung des Röntgengeräts befindet sich in der Steuer- und Verarbeitungseinheit 5. Der zu untersuchende Patient 6 liegt auf einem Patiententisch 7 so, daß ein Röntgenbild seines Kopfes erfaßt werden kann. An der Bildaufnahmeeinrichtung 4 sind seitlich zwei Infrarot-CCD-Kameras 8,9 angebracht, die die Detektoranordnung bilden. Die Kameras 8,9 sind so angebracht, daß deren Sichtbereich dem Durchleuchtungsbereich des Röntgengeräts 1, also dem vom Röntgenstrahler 3 durchleuchteten Bereich im wesentlichen entspricht. Die Kameras 8,9 können folglich Infrarotlichtsignale detektieren, die von drei am Patiententisch angeordneten Infrarot-Leuchtdioden emittiert werden. Die Leuchtdioden 10 sind derart am Patiententisch 7 angeordnet, daß sie nicht vom Kopf des Patienten 6 überdeckt werden. Weiterhin ist beispielhaft ein Behandlungsinstrument 11 im Durchleuchtungsbereich gezeigt, an dem ebenfalls drei Infrarot-Leuchtdioden 12 angebracht sind. Außerdem ist noch als Beispiel für ein weiteres medizinisches Gerät symbolisch ein Strahlentherapiegerät 13 gezeigt, das ebenfalls mit drei Infrarot-Leuchtdioden 14 ausgestattet ist. Zwar befindet sich dieses nicht im Durchleuchtungsbereich des Röntgengeräts 1, der Sichtbereich der Kameras 8,9 ist jedoch derart groß, daß auch Signale von den Leuchtdioden 14 detektiert werden können, wenn sich das Strahlentherapiegerät 13 nicht zu weit entfernt vom Patienten 6 befindet.

Die drei Infrarot-Leuchtdioden 10 am Patiententisch bilden eine Markeranordnung. Ebenso bilden die drei Leuchtdioden 12 am Behandlungsinstrument 11 und die drei Leuchtdioden 14 am Strahlentherapiegerät 13 eine Markeranordnung. Jede der beiden Kameras 8,9 kann die zweidimensionale Position einer Leuchtdiode erfassen. Aus den von den beiden Kameras 8,9 erfaßten Signalen einer Leuchtdiode kann die dreidimensionale Position einer Leuchtdiode bestimmt werden. Um die Position eines Geräts, beispielsweise des Patiententisches 7 in dreidimensionalen Koordinaten zu bestimmen, sind wie gezeigt pro Markeranordnung drei Leuchtdioden erforderlich.

Die Positionen werden zunächst im Detektorkoordinatensystem D bestimmt, das durch die Koordinatenachsen x_{D},y_{D},z_{D} gebildet wird. Das Detektorkoordinatensystem D ist fest mit der Detektoranordnung, im gezeigten Beispiel mit der Kamera 9 verkoppelt. Durch die Funktionalität des aus den Kameras 8,9 und Leuchtdioden 10,12,14 bestehenden Positionsmeßsystems können Positionen, die im Detektorkoordinatensystem D bestimmt wurden, auch leicht in Objektkoordinaten eines Objektkoordinatensystems O umgerechnet werden. Das durch die Koordinatenachsen x_{O},y_{O},z_{O} gebildete Objektkoordinatensystem O ist fest mit dem Untersuchungsobjekt, hier dem Patienten 6 bzw. fest mit dem Patiententisch 7, der bezüglich des Patienten 6 ortsfest ist, verkoppelt. Das Positionsmeßsystem erlaubt also die Erfassung der dreidimensionalen Position eines mit einer Markeranordnung ausgestatteten Geräts im Detetektorkoordinatensystem D und die Umrechnung dieser Position in Objektkoordinaten des Objektkoordinatensystems O.

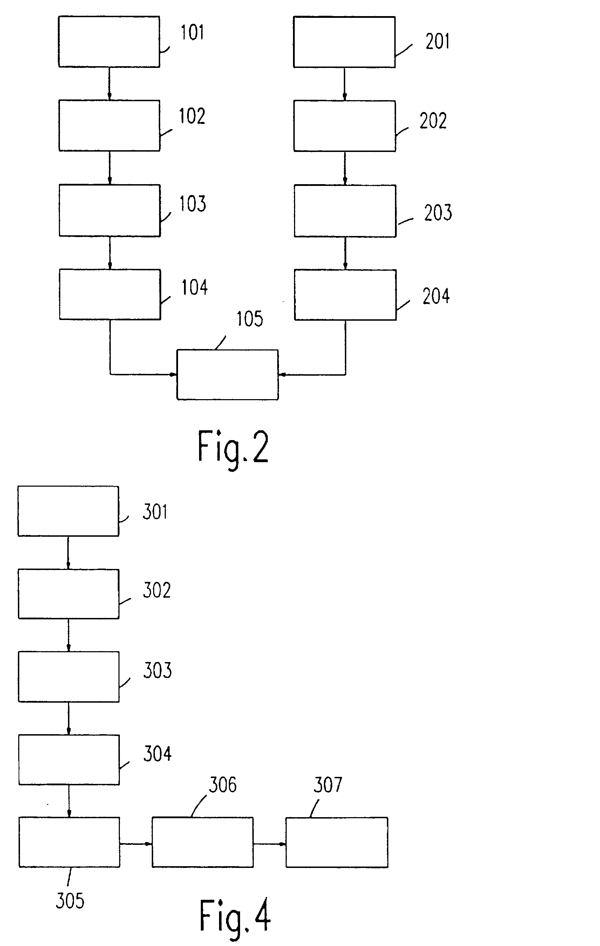

Anhand des in Fig. 2 gezeigten Ablaufplanes soll das Verfahren zur Bestimmung der Position eines als Bildpunkt in mindestens einem Röntgenbild abgebildeten Objektpunktes im Objektkoordinatensystem O näher erläutert werden. Zunächst wird in Block 101 in einer ersten Position des C-Bogens ein erstes Röntgenbild erfaßt. Gleichzeitig dazu wird mit den Kameras 8,9 die Position der Leuchtdioden 10 in Detektorkoordinaten erfaßt. Aus der Position der Leuchtdioden 10 ergibt sich gleichzeitig auch die Position des Patiententisches 7 und die Lage des Objektkoordinatensystems O (bzw. die Lage des Ursprungs des Objektkoordinatensystems O).

Im Block 102 wird anschließend die Position der Kameras 8,9 in Objektkoordinaten bestimmt, was durch die Funktionalität des Positionsmeßsystems und aufgrund der Kenntnis der Position des Objektkoordinatensystems O in Detektorkoordinaten einfach möglich ist.

In Block 103 wird danach die Position des Röntgengeräts 1 im Objektkoordinatensystem O bestimmt. Als charakteristische Punkte des Röntgengeräts 1 werden der Fokuspunkt im Röntgenstrahler 3 und die Abbildungsebene in der Bildaufnahmeeinrichtung 4 ausgewählt, deren Positionen in Objektkoordinaten bestimmt werden. Dazu werden die Positionen des Fokuspunktes und der Abbildungsebene von Detektorkoordinaten in Objektkoordinaten umgerechnet. Die Positionen in Detektorkoordinaten sind bei der Durchführung des erfindungsgemäßen Verfahrens als bekannt vorauszusetzen und werden beispielsweise aus einem einmalig für das Röntgengerät 1 durchzuführenden Eichvorgang mit Hilfe eines Eichphantoms gewonnen, was weiter unten näher erläutert werden soll.

Im Block 104 wird nun eine Gerade durch den Fokuspunkt und den Bildpunkt in der Abbildungsebene, dessen zugehöriger Objektpunkt im Objektkoordinatensystem bestimmt werden soll, gelegt. Auf dieser Geraden liegt der gesuchte Objektpunkt.

Eine genauere Bestimmung ist mit nur einem Röntgenbild nicht möglich. Soll die Position des Objektpunktes im Objektkoordinatensystem exakt bestimmt werden, so ist ein zweites Röntgenbild erforderlich, das in einer anderen Stellung des C-Bogens als bei der Erfassung des ersten Röntgenbildes erfaßt wird. In der zweiten Stellung des C-Bogens werden die in der ersten Stellung des C-Bogens durchgeführten Verfahrensschritte der Blöcke 101 bis 104 ebenfalls durchgeführt, was durch die Blöcke 201 bis 204 verdeutlicht wird. Als Ergebnis ergibt sich wiederum eine Gerade durch den Fokuspunkt des Röntgengeräts in der zweiten Stellung des C-Bogens und durch den Bildpunkt im zweiten Röntgenbild, dessen zugehöriger Objektpunkt bestimmt werden soll. Der Bildpunkt im ersten und im zweiten Röntgenbild sollte dazu möglichst zum selben Objektpunkt gehören.

Bei der Wahl der Bildpunkte in zwei unterschiedlichen Röntgenbildern kann der Benutzer unterstützt werden. Nach der Bestimmung des ersten Bildpunktes im ersten Röntgenbild kann die Verbindungsgerade zwischen dem ersten Bildpunkt und dem ersten Fokuspunkt bestimmt werden. Diese Verbindungsgerade kann auf das zweite Röntgenbild projiziert werden, wodurch die Information gewonnen wird, daß der zweite Bildpunkt (im zweiten Röntgenbild) auf dieser projizierten Gerade im zweiten Röntgenbild liegen muß. Wählt der Benutzer nun einen Bildpunkt auf dieser projizierten Gerade, kann nun die Verbindungsgerade zwischen dem gewählten zweiten Bildpunkt und dem zweiten Fokuspunkt bestimmt und diese wiederum auf das erste Röntgenbild projiziert werden. Der Benutzer kann dadurch iterativ in beiden Röntgenbildern die Position der gewählten Bildpunkte so lange variieren, bis ausreichende Genauigkeit erreicht ist.

Die Position des gesuchten Objektpunktes ergibt sich schließlich in Block 105 durch Berechnung des Schnittpunktes der beiden Geraden, die sich aus der Berechnung in den Blöcken 104 und 204 ergaben. Damit ist die exakte Position des Objektpunktes im Objektkoordinatensystem bekannt, der als Bildpunkt in zwei Röntgenbildern abgebildet ist. Dies kann beispielsweise dazu benutzt werden, einen Punkt, der in einem oder zwei Röntgenbildern abgebildet ist, im Patienten beispielsweise mit einem mit Leuchtdioden ausgestatteten Behandlungsinstrument wiederzufinden.

Im dreidimensionalen Raum müssen sich die beiden Gerade nicht notwendigerweise schneiden. Ergibt sich kein Schnittpunkt, so liegt der gesuchte Objektpunkt auf in der Mitte auf der kürzesten Verbindungsstrecke zwischen den beiden Geraden.

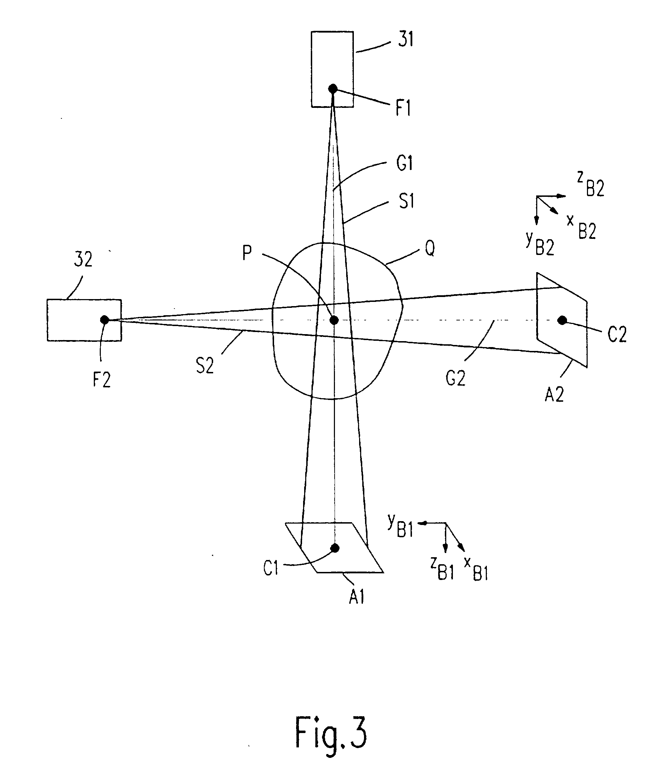

Das in Fig. 2 dargestellte Verfahren soll in Fig. 3 nochmals veranschaulicht werden. Mit Q ist ein Ausschnitt eines Untersuchungsobjektes bezeichnet, mit P ist der gesuchte Objektpunkt bezeichnet. In einer ersten Stellung des Röntgenstrahlers 31 wird das Untersuchungsobjekt Q mit einem ersten Strahlenbündel S1 durchleuchtet. Die Strahlung S1 geht dabei vom Fokuspunkt F1 des Röntgenstrahlers 31 aus und das Röntgenbild wird in Abbildungsebene A1 abgebildet. Der Objektpunkt P wird dabei beispielsweise als Bildpunkt C1 abgebildet, dessen Koordinaten im Bildkoordinatensystem des ersten Röntgenbildes (Koordinatenachsen x_{B1}, y_{B1}, z_{B1}) bekannt sind. In einer zweiten Stellung des Röntgenstrahlers 32 wird ein weiteres Röntgenbild erfaßt, das in der Abbildungsebene A2 abgebildet wird. Der Objektpunkt P wird dabei als Bildpunkt C2 im zweiten Bildkoordinatensystem (Koordinatenachsen x_{B2}, y_{B2}, z_{B2}) abgebildet.

Gemäß dem Verfahren wird jeweils eine Gerade durch die Punkte F1 und C1 (Gerade G1) bzw. F2 und C2 (Gerade G2) gelegt und deren Schnittpunkt berechnet. Dieser Schnittpunkt ist der gesuchte Objektpunkt P, der in den beiden Röntgenbildern als Bildpunkt C1 bzw. C2 abgebildet ist und dessen Position nun in Objektkoordinaten bekannt ist.

Wie leicht aus Fig. 3 zu erkennen ist, müssen die beiden Röntgenbilder nicht aus zueinander senkrechten Richtungen aufgenommen werden. Außerdem können auch mehr als zwei Röntgenbilder zur Positionsbestimmung verwendet werden.

Eine weitere Anwendungsmöglichkeit des erfindungsgemäßen Verfahrens soll durch das Ablaufdiagramm in Fig. 4 erläutert werden. Diese Anwendungsmöglichkeit besteht darin, die Position eines medizinischen Geräts zu bestimmen und beispielsweise in ein Röntgenbild einzublenden.

Die in den Blöcken 301 bis 303 symbolisch dargestellten Verfahrensschritte entsprechen den in Fig. 2 in den Blöcken 101 bis 103 dargestellten Verfahrensschritten, d.h., es wird ein Röntgenbild erfaßt und die Position von Fokus und Abbildungsebene des Röntgengeräts in Objektkoordinaten bestimmt. Im Block 304 wird die Position eines weiteren medizinischen Geräts, beispielsweise eines Behandlungsinstruments 11 oder eines Strahlentherapiegeräts 13 (siehe Fig. 1) mit den Kameras 8, 9 im Detektorkoordinatensystem D erfaßt. Bei dieser Erfassung muß entweder die Position des Röntgengeräts unverändert sein gegenüber der Position, in der in das Röntgenbild erfaßt wurde, oder es muß die erste Position gespeichert sein. Der in Block 304 erfolgte Schritt kann auch gleichzeitig zu den Verfahrensschritten der Blöcke 301 bis 303 erfolgen. Die Detektorkoordinaten des medizinischen Geräts werden anschließend im Block 305 in Objektkoordinaten umgerechnet. Um die Position des Gerätes in Bildkoordinaten zu bestimmen und diese Position im Röntgenbild einzublenden, wird im Block 306 zunächst eine Gerade durch den Fokuspunkt des Röntgenstrahlers und die Position des Gerätes im Objektkoordinatensystem gezogen, welche die Abbildungsebene schneidet. In Block 307 wird dann dieser Schnittpunkt berechnet, woraus sich die Position des Gerätes im Bildkoordinatensystem des Röntgenbildes ergibt.

Falls die Position des Röntgenstrahlers bei der Erfassung der Position des medizinischen Geräts eines andere war als die Position bei der Röntgenbildaufnahme, kann die Position des medizinischen Geräts von Objektkoordinaten mithilfe der gespeicherten Positon des Röntgengeräts in Detektorkoordinaten des Röntgengeräts bei der Röntgenbildaufnahme umgerechnet. Daraus kann dann die Position des Gerätes im Bildkoodinatensystem des Röntgenbildes wie oben beschrieben bestimmt werden.

Das in Fig. 4 beschriebene Verfahren wird beispielsweise dazu verwendet, um die Position eines chirurgischen Instruments, das innerhalb des Patienten geführt wird und das bis auf seine Leuchtdiodenanordnung von außen nicht sichtbar ist, im Röntgenbild anzuzeigen bzw. das Instrument selbst einzublenden. Dies kann sowohl bei abgeschaltetem Röntgengerät erfolgen, wenn die Röntgenbilder vorab erstellt wurden, als auch bei angeschaltetem Röntgenbild und ständiger Röntgendurchleuchtung. wenn beispielsweise eine im Körper geführte Biopsienadel im Röntbild nicht sichtbar ist.

Die Position des Instruments kann auch laufend aktualisiert werden, indem die in den Blöcken 304 bis 307 beschriebenen Verfahrensschritte laufend wiederholt werden.

Für den Fall, daß die Position eines Strahlentherapiegeräts laufend mit dem Positionsmeßsystem bestimmt wird, kann dies dazu verwendet werden, den Therapiestrahl exakt auf eine vorbestimmte Stelle, die beispielsweise anhand eines Röntgenbildes ausgewählt wird, zu positionieren.

Zur Bestimmung der Position des Fokuspunktes und der Lage der Abbildungsebene im Detektorkoordinatensystem ist für ein Röntgengerät einmalig, beispielsweise bei der Installation eines beschriebenen Positionsmeßsystems, ein Eichvorgang erforderlich. Dazu wird ein Eichphantom quasi als Untersuchungsobjekt in den Sichtbereich der Kameras und den Durchleuchtungsbereich des Röntgengeräts gebracht. Dieses Eichphantom ist mit einer ersten, Röntgenstrahlung absorbierenden Markeranordnung und mit einer zweiten Markeranordnung für die Kameras, beispielsweise Infrarot-Leuchtdioden bei Verwendung von Infrarot-Kameras. versehen. Die relative Lage der beiden Markeranordnungen zueinander ist dabei bekannt. Anschließend wird gleichzeitig sowohl mit dem Röntgengerät ein Röntgenbild des Eichphantoms, in dem die erste Markeranordnung mit abgebildet wird, als auch mit den Kameras die Position der zweiten Markeranordnung erfaßt. Die Position der zweiten Markeranordnung (der Leuchtdioden) in Detektorkoordinaten ist damit unmittelbar bekannt, woraus sich auch die Position der ersten Markeranordnung in Detektorkoordinaten sehr leicht berechnen läßt, da die relative Lage der beiden Markeranordnungen zueinander bekannt ist. Bei Auswahl eines geeigneten Eichphantoms mit in geeigneter Weise angeordneten Markeranordnungen kann mit Hilfe geometrischer Überlegungen aus der nun bekannten Position der ersten Markeranordnung und deren Abbildung im Röntgenbild sehr leicht die Position des Fokuspunktes des Röntgengeräts und die Lage der Abbildungsebene des Röntgengeräts berechnet werden. Sofern die Anordnung der Kameras am Röntgengerät später nicht mehr verändert wird, bleiben diese Werte quasi als Fixwerte für das Röntgengerät unverändert und können als bekannt für das erfindungsgemäße Verfahren vorausgesetzt und benutzt werden.

Um die Genauigkeit der Positionsbestimmung weiter zu erhöhen oder für den Fall, daß die Sichtlinien der Kameras verdeckt werden könnten, kann zusätzlich eine weitere Detektoranordnung, die beispielsweise wie im bekannten Fall an einem Stativ angebracht ist, verwendet werden.

Auch für diagnostische Zwecke kann das erfindungsgemäße Verfahren und die erfindungsgemäße Anordnung verwendet werden. So ist beispielsweise die positionsgenaue Kombination mehrerer Röntgenbilder, die bei verschiedenen Positionen des Röntgentisches aufgenommen wurden, zu einem einzigen Röntgenbild möglich. Dazu werden beispielsweise der Fokus und die Abbildungsebene festgehalten und der Patiententisch wird bewegt, wobei in zwei verschiedenen Positionen des Patiententisches jeweils ein Röntgenbild aufgenommen wird. Die Position des Patiententisches ist dabei über die am Patiententisch oder am Patienten angeordnete Markeranordnung feststellbar.

Die Erfindung ist nicht auf die in den Zeichnungen gezeigte Anwendung bei einem C-Bogen-Röntgengerät beschränkt. Es können auch mit anderen Röntgengeräten Röntgenbilder aus unterschiedlichen Positionen bezüglich eines Untersuchungsobjekts gewonnen werden, beispielsweise mit einem schwenkbaren Patiententisch mit fest angeordnetem Röntgenstrahler und Bildaufnehmer. Bei derartigen Röntgengeräten könnten beispielsweise die Kameras am Röntgenstrahler und die Leuchtdioden am schwenkbaren Tisch angeordnet sein. Auch bei zunehmend verwendeten mobilen Röntgen- oder Computertomographieanlagen kann die Erfindung eingesetzt werden. Bei einer CT-Anlage können die Kameras an der Abtasteinheit (Gantry) und die Leuchtdioden am Patiententisch angebracht sein.

## Patentansprüche

1. Verfahren zur Positionsbestimmung bei der Röntgenbildgebung, bei dem
- mit einem Röntgengerät (1), das einen Röntgenstrahler (3) und eine Röntgenbildaufnahme- oder Röntgenbildverstärkereinrichtung (4) umfasst, mindestens ein Röntgenbild eines Untersuchungsobjektes (6) erstellt wird und mit einer am Röntgengerät (1) angeordneten Detektoranordnung (8, 9) die Position einer am Untersuchungsobjekt (6) oder relativ zum Untersuchungsobjekt (6) ortsfest angeordneten Markeranordnung (10) in einem mit der Detektoranordnung (8, 9) verkoppelten Detektorkoordinatensystem (D) erfaßt wird,
- die Position des Röntgengerätes (1) in einem mit dem Untersuchungsobjekt (6) verkoppelten Objektkoordinatensystem (O) bestimmt wird
- und danach die Position eines als Bildpunkt (C1, C2) in einem Röntgenbild abgebildeten Objektpunktes (P) im Objektkoordinatensystem (O) bestimmt wird.

2. Verfahren nach Anspruch 1,
**dadurch gekennzeichnet, daß** mindestens zwei Röntgenbilder des Untersuchungsobjektes (6) aus unterschiedlichen Richtungen erfaßt werden und daß gleichzeitig zur Röntgenbilderfassung die Position des Fokuspunktes (F1, F2) und die Lage der Abbildungsebene (A1, A2) des Röntgengerätes im Objektkoordinatensystem (O) bestimmt werden.

3. Verfahren nach Anspruch 2,
**dadurch gekennzeichnet, daß** die Position des Objektpunktes (P) mittels zweier Geraden (G1, G2) bestimmt wird, wobei die erste Gerade (G1) durch den zum Objektpunkt (P) gehörigen Bildpunkt (C1) in einem ersten Röntgenbild und durch die erste Position des Fokuspunktes (F1) verläuft und die zweite Gerade (G2) durch den zum Objektpunkt (P) gehörigen Bildpunkt (C2) in einem zweiten Röntgenbild und durch die zweite Position des Fokuspunktes (F2) verläuft, wobei sich der Objektpunkt (P) als Schnittpunkt der beiden Geraden (G1, G2) oder als Mittelpunkt der kürzesten Verbindungsstrecke der beiden Geraden (G1, G2) ergibt.

4. Verfahren nach einem der vorhergehenden Ansprüche,
**dadurch gekennzeichnet, daß** mit der Detektoranordnung (8, 9) die Position eines mit einer weiteren Markeranordnung (12) versehenen medizinischen Gerätes (11) im Detektorkoordinatensystem (D) erfaßt wird und daß aus der Position des medizinischen Gerätes (11) im Detektorkoordinatensystem (D) dessen Position im Bildkoordinatensystem (B) eines Röntgenbildes bestimmt wird.

5. Verfahren nach Anspruch 4,
**dadurch gekennzeichnet, daß** die Position des medizinischen Gerätes (11) im Bildkoordinatensystem (B) aus dem Schnittpunkt einer durch den Fokuspunkt (F1, F2) und die Position des medizinischen Gerätes (11) im Objektkoordinatensystem (O) verlaufenden Gerade mit der Abbildungsebene (A1, A2) berechnet wird.

6. Anordnung zur Durchführung des Verfahrnes nach Anspruch 1 mit einem Röntgengerät (1), welches einen Röntgenstrahler (3) und eine Röntgenbildaufnahme- oder Röntgenbildverstärkereinrichtung (4) umfasst, mit einer Detektoranordnung mit mindestens zwei Detektorelementen (8,9) und mit einer Markeranordnung (10),
**gekennzeichnet dadurch,**
**dass** die Detektoranordnung (8, 9) am Röntgengerät (1) angeordnet ist,
**dass** die Markeranordnung (10) am Untersuchungsobjekt (6) oder relativ zum Untersuchungsobjekt (6) ortsfest angeordnet ist und
**dass** die Detektoranordnung dazu vorgesehen ist, die Position der Markeranordnung (10) in einem mit der Detektoranordnung verkoppelten Detektorkoordinatensystem (D) zu erfassen.

7. Anordnung nach einem der Anspruch 6,
**dadurch gekennzeichnet, daß** die Detektoranordnung (8, 9) am Röntgenstrahler (3) oder an der Röntgenbildaufnahme- oder Röntgenbildverstärkereinrichtung (4) angeordnet ist.

8. Anordnung nach einem der Ansprüche 6 oder 7,
**dadurch gekennzeichnet, daß** eine weitere Markeranordnung (12, 14) an einem medizinischen Gerät, insbesondere einem Behandlungsinstrument (11) oder einer Strahlentherapieanordnung (13) angeordnet ist.

9. Anordnung nach einem der Ansprüche 6 bis 8,
**dadurch gekennzeichnet, daß** die Markeranordnungen (10, 12, 14) optische Lichtquellenmarker, beispielsweise Infrarotlicht oder sichtbares Licht emittierende Leuchtdioden, retroreflektierende Marker oder mit einem Erkennungsmuster versehene Marker aufweisen.

10. Anordnung nach einem der Ansprüche 6 bis 9,
**dadurch gekennzeichnet, daß** die Detektoranordnung (8, 9) zwei, jeweils eine zweidimensionale Position erfassende oder drei, jeweils eine eindimensionale Position erfassende optische Detektoren umfaßt, welche Infrarotlicht oder sichtbares Licht detektieren können, vorzugsweise Infrarot-CCD-Kameras, und daß die Markeranordnungen (10, 12, 14) mindestens jeweils drei Infrarotlicht oder sichtbares Licht emittierende Leuchtdioden umfassen.

11. Anordnung nach einem der Ansprüche 6 bis 8,
**dadurch gekennzeichnet, daß** die Markeranordnung mindestens einen elektromagnetischen Sendeanordnung und die Detektorvorrichtung mindestens eine elektromagnetische Empfangsanordnung aufweist.

12. Anordnung nach einem der Ansprüche 6 bis 11,
**dadurch gekennzeichnet, daß** das Röntgengerät (1) ein C-Bogen-Röntgengerät ist.

## Claims

1. A method of position determination X-ray imaging, in which
- at least one X-ray image of an object (6) to be examined is formed by means of an X-ray apparatus (1), which comprises an X-ray source (3) and an X-ray pick-up or X-ray intensifier device (4) and with a detector device (8, 9) mounted on the X-ray apparatus (1) the position of a marking device (10), provided so as to be stationary on the object (6) to be examined or stationary relative to the object (6) to be examined is determined in a detector co-ordinate system (D) coupled to the detector device (8, 9),
- the position of the X-ray apparatus (1) is determined in an object co-ordinate system (O), which is coupled to the object (6) to be examined,
- and, subsequently, the position of an object point (P) which is imaged as an image point (C1, C2) in an X-ray image, is determined in the object co-ordinate system (O).

2. A method as claimed in Claim 1, **characterized in that** at least two X-ray images of the object (6) to be examined are formed from different directions, the position of the focal point (F1, F2) and the position of the imaging plane (A1, A2) of the X-ray apparatus in the object co-ordinate system (O) being determined simultaneously with the X-ray image acquisition.

3. A method as claimed in Claim 2, **characterized in that** the position of the object point (P) is determined by means of two straight lines (G1, G2), the first straight line (G1) extending through the image point (C1) associated with the object point (P) in a first X-ray image and through the first position of the focal point (F1), whereas the second straight line (G2) extends through the image point (C2) associated with the object point (P) in a second X-ray image and through the second position of the focal point (F2), the object point (P) being the point of intersection of the two straight lines (G1, G2) or the central point of the shortest connecting line between the two straight lines (G1, G2).

4. A method as claimed in one of the preceding Claims, **characterized in that** the detector device (8, 9) detects the position of a medical apparatus (11) provided with a further marking device (12), in the detector co-ordinate system (D), the position of the medical apparatus (11) in the image co-ordinate system (B) of an X-ray image being determined from its position in the detector co-ordinate system (D).

5. A method as claimed in Claim 4, **characterized in that** the position of the medical apparatus (11) in the image co-ordinate system (B) is calculated from the point of intersection between the imaging plane (A1, A2) and a straight line which extends through the focal point (F1, F2) and the position of the medical apparatus (11) in the object coordinate system (O).

6. A system for carrying out the method as claimed in Claim 1, including an X-ray apparatus (1) which comprises an X-ray source (3) and an X-ray pick-up or X-ray intensifier device (4) having a detector device with at least two detector elements (8, 9) and a marking device (10), **characterized in that** the detector device (8, 9) is mounted on the X-ray apparatus (1) and that the marking device (10) is provided so as to be stationary on the object (6) to be examined or stationary relative to the object (6) to be examined, the detector device (8, 9) being designed such that it detects the position of the marking device (10) in a detector coordinate system (D) coupled to the detector device.

7. A system as claimed in Claim 6, **characterized in that** the detector device (8, 9) is mounted on the X-ray source (3) or on the X-ray image pick-up or X-ray image intensifier device (4).

8. A system as claimed in one of the Claims 6 or 7, **characterized in that** a further marking device (12, 14) is mounted on a medical apparatus, notably a treatment instrument (11) or a radiation therapy device (13).

9. A system as claimed in one of the Claims 6 to 8, **characterized in that** the marking devices (10, 12, 14) include optical light source markers, for example light emitting diodes which emit infrared light or visible light, retro-reflective markers or markers provided with a recognition pattern.

10. A system as claimed in one of the Claims 6 to 9, **characterized in that** the detector device (8, 9) includes two optical detectors, detecting each a two-dimensional position, or three optical detectors, detecting each a one-dimensional position, said detectors being capable of detecting infrared light or visible light and preferably being infrared CCD cameras, each of the marking devices (10, 12, 14) including at least three light emitting diodes emitting infrared light or visible light.

11. A system as claimed in one of the Claims 6 to 8, **characterized in that** the marking device includes at least one electromagnetic transmitter device, the detector device including at least one electromagnetic receiver device.

12. A system as claimed in one of the Claims 6 to 11, **characterized in that** the X-ray apparatus (1) is a C-arm X-ray apparatus.

## Revendications

1. Procédé pour déterminer la position pendant l'imagerie radiologique, dans lequel
- au moins une image radiographique d'un objet à examiner (6) est établie avec un appareil radiographique (1) qui comprend un générateur de rayons X (3) et un dispositif enregistreur ou amplificateur d'images radiographiques et avec un dispositif détecteur (8,9) disposé sur l'appareil radiographique (1), la position d'un dispositif marqueur (10) disposé sur l'objet à examiner (6) ou de manière stationnaire par rapport à l'objet à examiner (6) est enregistrée dans un système de coordonnées du détecteur (D) couplé au dispositif détecteur (8, 9),
- la position de l'appareil radiographique (1) est déterminée dans un système de coordonnées d'objet (O) couplé à l'objet à examiner (6)
- et, ensuite, la position d'un point d'objet (P) représenté comme point d'image (C1, C2) dans une image radiographique est déterminée dans le système de coordonnées d'objet (O).

2. Procédé selon la revendication 1,
**caractérisé en ce qu'**au moins deux images radiographiques de l'objet à examiner (6) sont enregistrées à partir de directions différentes et que la position du point de foyer (F1, F2) et la position du plan de représentation (A1, A2) de l'appareil radio graphique dans le système de coordonnées d'objet (O) sont déterminées simultanément pour l'enregistrement de l'image radiographique.

3. Procédé selon la revendication 2,
**caractérisé en ce que** la position du point d'objet (P) est déterminée à l'aide de deux droites (G1, G2), la première droite (G1) s'étendant par le point d'image (C1) appartenant au point d'objet (P) dans une première image radiographique et par la première position du point de foyer (F1) et la deuxième droite (G2) s'étendant par le point d'image (C2) appartenant au point d'objet (P) dans une deuxième image radiographique et par la deuxième position du point de foyer (F2), le point d'objet (P) étant obtenu comme l'intersection des deux droites (G1, G2) ou comme point central du tronçon de jonction le plus court des deux droites (G1, G2).

4. Procédé selon l'une des revendications précédentes,
**caractérisé en ce que** la position d'un appareil médical (11) doté d'un dispositif marqueur (12) supplémentaire est enregistrée avec le dispositif détecteur (8, 9) dans le système de coordonnées du détecteur (D) et qu'à partir de la position de l'appareil médical (11) dans le système de coordonnées du détecteur (D), sa position est déterminée dans le système de coordonnées d'image (B) d'une image radiographique.

5. Procédé selon la revendication 4,
**caractérisé en ce que** la position de l'appareil médical (11) dans le système de coordonnées de l'image (B) est calculée à partir de l'intersection d'une droite s'étendant par le point de foyer (F1, F2) et la position de l'appareil médical (11) dans le système de coordonnées de l'objet (O) avec le plan de représentation (A1, A2).

6. Dispositif de mise en oeuvre du procédé selon la revendication 1 avec un appareil radiographique (1) qui comprend un générateur de rayons X (3), un dispositif enregistreur ou amplificateur d'images radiographiques (4) avec un dispositif détecteur doté d'au moins deux éléments détecteurs (8, 9) et avec un dispositif marqueur (10),
**caractérisé en ce que** le dispositif détecteur (8, 9) est disposé sur l'appareil radiographique (1) et que le dispositif marqueur (10) est disposé sur l'objet à examiner (6) ou de manière stationnaire par rapport à l'objet à examiner (6), le dispositif détecteur (8, 9) étant conçu de telle sorte qu'il enregistre la position du dispositif marqueur (10) dans un système de coordonnées du détecteur (D) couplé au dispositif détecteur.

7. Dispositif selon la revendication 6,
**caractérisé en ce que** le dispositif détecteur (8, 9) est disposé sur le générateur de rayons X (3) ou sur le dispositif enregistreur ou amplificateur d'image (4).

8. Dispositif selon l'une des revendications 6 ou 7,
**caractérisé en ce qu'**un dispositif marqueur (12, 14) supplémentaire est disposé sur un appareil médical, en particulier un instrument de traitement (11) ou un dispositif thérapeutique aux rayons X (13).

9. Dispositif selon l'une des revendications 6 à 8,
**caractérisé en ce que** les dispositifs marqueurs (10, 12, 14) présentent des marqueurs optiques à source lumineuse, par exemple des diodes électroluminescentes émettant par exemple de la lumière infrarouge ou de la lumière visible, des marqueurs rétroréfléchissants ou des marqueurs dotés d'un modèle de reconnaissance.

10. Dispositif selon l'une des revendications 6 à 9,
**caractérisé en ce que** le dispositif détecteur (8, 9) comporte deux détecteurs optiques enregistrant respectivement une position bidimensionnelle ou trois détecteurs optiques enregistrant respectivement une position unidimensionnelle qui peuvent détecter de la lumière infrarouge ou de la lumière visible, de préférence une caméra CCD à infrarouges, et que les dispositifs marqueurs (10, 12, 14) comprennent respectivement au moins trois diodes électroluminescentes émettant de la lumière infrarouge ou de la lumière visible.

11. Dispositif selon l'une des revendications 6 à 8,
**caractérisé en ce que** le dispositif marqueur comprend au moins un dispositif de transmission électromagnétique et le dispositif détecteur au moins un dispositif de réception électromagnétique.

12. Dispositif selon l'une des revendications 6 à 11,
**caractérisé en ce que** l'appareil radiographique (1) est un arc radiographique.
